# EUROPEAN PATENT APPLICATION

(11) **EP 1 916 000 A1**
(43) Date of publication of application: **30.04.2008**
(21) Application number: 06291676.2
(22) Date of filing: 26.10.2006
(51) Int. Cl.: A61K 39/165, C07K 14/12

(54) **Use of measles virus nucleoprotein for treating atherosclerosis**

(71) Applicant: INSERM (Institut National de la Santé et de la Recherche Médicale), 75654 Paris Cedex 13 (FR)
(72) Inventor: The designation of the inventor has not yet been filed
(74) Representative: Cabinet Plasseraud

(57) **Abstract**

The present invention relates to the use of the measles virus nucleoprotein (NP), fragments or variants thereof for the treatment and/or the prevention of atherosclerosis.

SEQUENCE LISTING

<110>  I.N.S.E.R.M

<120>  Use of measles virus nucleoprotein for treating atherosclerosis

<130>  BEP060428 EP

<160>  2

<170>  PatentIn version 3.3

<210>  1
<211>  525
<212>  PRT
<213>  Measles virus

<400>  1

Met Ala Thr Leu Leu Arg Ser Leu Ala Leu Phe Lys Arg Asn Lys Asp
1               5                   10                  15

Lys Pro Pro Ile Thr Ser Gly Ser Gly Gly Ala Ile Arg Gly Ile Lys
            20                  25                  30

His Ile Ile Ile Val Pro Ile Pro Gly Asp Ser Ser Ile Thr Thr Arg
        35                  40                  45

Ser Arg Leu Leu Asp Arg Leu Val Arg Leu Ile Gly Asn Pro Asp Val
    50                  55                  60

Ser Gly Pro Lys Leu Thr Gly Ala Leu Ile Gly Ile Leu Ser Leu Phe
65                  70                  75                  80

Val Glu Ser Pro Gly Gln Leu Ile Gln Arg Ile Thr Asp Asp Pro Asp
                85                  90                  95

Val Ser Ile Arg Leu Leu Glu Val Val Gln Ser Asp Gln Ser Gln Ser
            100                 105                 110

Gly Leu Thr Phe Ala Ser Arg Gly Thr Asn Met Glu Asp Glu Ala Asp
        115                 120                 125

Gln Tyr Phe Ser His Asp Asp Pro Ile Ser Ser Asp Gln Ser Arg Phe
    130                 135                 140

Gly Trp Phe Glu Asn Lys Glu Ile Ser Asp Ile Glu Val Gln Asp Pro
145                 150                 155                 160

Glu Gly Phe Asn Met Ile Leu Gly Thr Ile Leu Ala Gln Ile Trp Val
                165                 170                 175

Leu Val Ala Lys Ala Val Thr Ala Pro Asp Thr Ala Ala Asp Ser Glu
            180                 185                 190

Leu Arg Arg Trp Ile Lys Tyr Thr Gln Gln Arg Arg Val Val Gly Glu

        195                 200                 205

Phe Arg Leu Glu Arg Lys Trp Leu Asp Val Val Arg Asn Arg Ile Ala
    210                 215                 220

Glu Asp Leu Ser Leu Arg Arg Phe Met Val Ala Leu Ile Leu Asp Ile
225                 230                 235                 240

Lys Arg Thr Pro Gly Asn Lys Pro Arg Ile Ala Glu Met Ile Cys Asn
                245                 250                 255

Ile Asp Thr Tyr Ile Val Glu Ala Gly Leu Ala Ser Phe Ile Leu Thr
            260                 265                 270

Ile Lys Phe Gly Ile Glu Thr Met Tyr Pro Ala Leu Gly Leu His Glu
        275                 280                 285

Phe Asp Gly Glu Leu Ser Thr Leu Glu Ser Leu Met Asn Leu Tyr Gln
    290                 295                 300

Gln Met Gly Glu Thr Ala Pro Tyr Met Val Ile Leu Glu Asn Ser Ile
305                 310                 315                 320

Gln Asn Lys Phe Ser Ala Gly Ser Tyr Pro Leu Leu Trp Ser Tyr Ala
                325                 330                 335

Met Gly Val Gly Val Glu Leu Glu Asn Ser Met Gly Gly Leu Asn Phe
            340                 345                 350

Gly Arg Ser Tyr Phe Asp Pro Ala Tyr Phe Arg Leu Gly Gln Glu Met
        355                 360                 365

Val Arg Arg Ser Ala Gly Lys Val Ser Ser Thr Leu Ala Ser Glu Leu
    370                 375                 380

Gly Ile Thr Ala Glu Asp Ala Arg Leu Val Ser Glu Ile Ala Met His
385                 390                 395                 400

Thr Thr Glu Asp Lys Ile Ser Arg Ala Val Gly Pro Arg Gln Ala Gln
                405                 410                 415

Val Ser Phe Leu His Gly Asp Gln Ser Glu Asn Glu Leu Pro Arg Leu
            420                 425                 430

Gly Gly Lys Glu Asp Arg Arg Val Lys Gln Ser Arg Gly Glu Ala Arg
        435                 440                 445

Glu Ser Tyr Arg Glu Thr Gly Pro Ser Arg Ala Ser Asp Ala Arg Ala
    450                 455                 460

Ala His Leu Pro Thr Gly Thr Pro Leu Asp Ile Asp Thr Ala Ser Glu

465                 470                 475                 480

Ser Ser Gln Asp Pro Gln Asp Ser Arg Arg Ser Ala Asp Ala Leu Leu
                485                 490                 495

Arg Leu Gln Ala Met Ala Gly Ile Ser Glu Glu Gln Gly Ser Asp Thr
            500                 505                 510

Asp Thr Pro Ile Val Tyr Asn Asp Arg Asn Leu Leu Asp
        515                 520                 525

<210>  2
<211>  125
<212>  PRT
<213>  Measles virus

<400>  2

Thr Thr Glu Asp Lys Ile Ser Arg Ala Val Gly Pro Arg Gln Ala Gln
1               5                   10                  15

Val Ser Phe Leu His Gly Asp Gln Ser Glu Asn Glu Leu Pro Arg Leu
            20                  25                  30

Gly Gly Lys Glu Asp Arg Arg Val Lys Gln Ser Arg Gly Glu Ala Arg
        35                  40                  45

Glu Ser Tyr Arg Glu Thr Gly Pro Ser Arg Ala Ser Asp Ala Arg Ala
    50                  55                  60

Ala His Leu Pro Thr Gly Thr Pro Leu Asp Ile Asp Thr Ala Ser Glu
65                  70                  75                  80

Ser Ser Gln Asp Pro Gln Asp Ser Arg Arg Ser Ala Asp Ala Leu Leu
                85                  90                  95

Arg Leu Gln Ala Met Ala Gly Ile Ser Glu Glu Gln Gly Ser Asp Thr
            100                 105                 110

Asp Thr Pro Ile Val Tyr Asn Asp Arg Asn Leu Leu Asp
        115                 120                 125

## Description

The present invention relates to the treatment and/or the prevention of atherosclerosis.

Atherosclerosis is the most common cause of death in western societies and is predicted to become the leading cause of cardiovascular disease in the world within two decades.

Atherosclerosis can be considered to be a form of chronic inflammation resulting from interaction between modified lipoproteins, monocyte-derived macrophages, T cells and the normal cellular elements of the arterial wall. Actually, atherosclerosis develops in response to arterial wall injury caused by several agents, namely modified lipids (Binder et al., 2002; Hansson et al., 2006), and ultimately leads to the development of atherosclerotic vascular diseases (AVD) which may affect the coronary arteries (causing ischaemic heart disease), the cerebral circulation (causing cerebrovascular disease), the aorta (producing aneurysms that are prone to thrombosis and rupture) and peripheral blood vessels, typically the legs (causing peripheral vascular disease and intermittent claudication). Ischaemic heart disease (IHD) includes angina (chest pain caused by insufficient blood supply to cardiac muscle) and myocardial infarction (death of cardiac muscle) and cerebrovascular disease includes stroke and transient ischaemic attacks.

Immune pathogenic and regulatory responses control the development and progression of atherosclerosis (Binder et al., 2002; Hansson et al., 2006; Tegdui et al., 2006). Whereas early lesion development appears to be driven by a T helper cell type 1 (Th1) response (Gupta et al., 1997; Whitman et al., 2000), the progression of advanced lesions may also be promoted under a Th2-biased immune environment (Davenport et al., 2000). This highlights the need to control both Th1 and Th2-driven responses in order to ensure efficient and sustained protection against atherosclerosis. Recently, it has been shown that defined populations of naturally occurring or antigen-induced regulatory T cells, known to suppress both Th1 and Th2 responses (Sakaguchi et al., 2005; Maloy et al., 2001), greatly contribute to inhibition of lesion development in murine models of atherosclerosis (Mallat et al., 2003; Ait-Oufella et al., 2006), in part through the production of transforming growth factor-b (Robertson et al., 2004; Ait-Oufella et al., 2006) and interleukin (II)-10 (Mallat et al., 2003).

Thus, strategies promoting an endogenous regulatory immune response represents a major interest in developing novel therapies to treat atherosclerosis.

For instance, document WO2006072888 describes a composition or a patch adapted for the prophylactic or therapeutic treatment by continuous subcutaneous administration of a subject suffering from atherosclerosis, comprising an effective amount of at least one epitope derived from a protein present in the atherosclerotic plaque. The administration of said epitope induces a specific regulatory immune response.

The purpose of the present invention is to provide methods and compositions for generating a regulatory T cell response, within the atherosclerotic lesion sites to prevent unwanted Th1/Th2 pro-atherogenic immunity.

The present invention now surprisingly shows that administration of measles virus nucleoprotein (NP), variants or fragments thereof, to mice susceptible to atherosclerosis significantly reduces the development of new atherosclerotic lesions and markedly inhibits the progression of established plaques by promoting an anti-inflammatory T regulatory cell type 1-like response. These findings identify a novel mechanism of immune modulation by measles virus nucleoprotein and indicate that measles virus nucleoprotein (NP), variants or fragments thereof may be used for the treatment and/or the prevention of atherosclerosis, the first cause of heart disease and stroke.
Thus, an aspect of the invention relates to the use of a polypeptide comprising the amino acid sequence as set forth in SEQ ID NO:2 for the manufacture of a medicament for the treatment and/or the prevention of atherosclerosis.
SEQ ID NO:2 corresponds to the amino acid sequence ranging from positions 400 to 525 of SEQ ID N0:1.
Another aspect of the invention relates to the use of a polypeptide comprising an amino acid sequence having at least 80% identity with SEQ ID NO:2, said amino acid sequence having anti-atherosclerosis activity, for the manufacture of a medicament for the treatment and/or the prevention of atherosclerosis.
Typically, the amino acid sequence has at least 85%, preferably 90% and even more preferably 95% identity with SEQ ID NO:2.
Another aspect of the invention relates to the use of a polypeptide comprising a fragment of the amino acid sequence described above, said fragment having anti-atherosclerosis activity, for the manufacture of a medicament for the treatment and/or the prevention of atherosclerosis.
Typically, the fragment of the amino acid sequence has at least 30, 50, 60 or 70 amino acids, preferably at least 80, 90 or 100 amino acids and even more preferably at least 110 or 120 amino acids.
Typically a polypeptide according to the invention may have at most 600, 525, 500, 400, 300 or 125 amino acids.
Typically a polypeptide according to the invention may have at least 30, 50, 60 or 70 amino acids, preferably at least 80, 90 or 100 amino acids and even more preferably at least 110 or 120 amino acids.
Typically, the invention relates to an use as described above, wherein said polypeptide comprises the amino acid sequence as set forth in SEQ ID NO:1 or an amino acid sequence having at least 70% identity with the amino acid sequence as set forth in SEQ ID NO:1. Typically, the amino acid sequence may have at least 80%, preferably 90% and even more preferably 95% identity with SEQ ID NO:1.
The anti-athersclerosis activity may be assessed according to any of the method described in the examples.
Another aspect of the invention relates to the use of a nucleic acid encoding a polypeptide as described above, for the manufacture of a medicament for the treatment and/or the prevention of atherosclerosis.

Another aspect of the invention relates to a method for treating and/or preventing atherosclerosis comprising administering to a subject in need thereof a therapeutically effective amount of polypeptide or nucleic acid, as described above. Typically the administration may be repeated.

### Definitions :

A "coding sequence" or a sequence "encoding" an expression product, such as an RNA, polypeptide, protein, or enzyme, is a nucleotide sequence that, when expressed, results in the production of that RNA, polypeptide, protein, or enzyme, i.e. the nucleotide sequence encodes an amino acid sequence for that polypeptide, protein or enzyme. A coding sequence for a protein may include a start codon (usually ATG) and a stop codon.

As used herein, references to specific proteins (e.g. measles virus nucleoprotein (NP)) can include a polypeptide having a native amino acid sequence, as well as variants and modified forms regardless of their origin or mode of preparation. A protein that has a native amino acid sequence is a protein having the same amino acid sequence as obtained from nature (e.g., a naturally occurring NP). Such native sequence proteins can be isolated from nature or can be prepared using standard recombinant and/or synthetic methods. Native sequence proteins specifically encompass naturally occurring truncated or soluble forms, naturally occurring variant forms (e.g., alternatively spliced forms), naturally occurring allelic variants and forms including postranslational modifications. A native sequence protein includes proteins following post-translational modifications such as glycosylation, or phosphorylation, or other modifications of some amino acid residues.

"Function-conservative variants" refer to proteins that are functional equivalents to a native sequence protein that have similar amino acid sequences and retain, to some extent, one or more activities of the native protein. Variants also include fragments that retain activity. Variants also include proteins that are substantially identical (e.g., that have 80, 85, 90, 95, 97, 98, 99%, sequence identity) to a native sequence. Such variants include proteins having amino acid alterations such as deletions, insertions and/or substitutions. A "deletion" refers to the absence of one or more amino acid residues in the related protein. The term "insertion" refers to the addition of one or more amino acids in the related protein. A "substitution" refers to the replacement of one or more amino acid residues by another amino acid residue in the polypeptide. Typically, such alterations are conservative in nature such that the activity of the variant protein is substantially similar to a native sequence protein (see, e.g., Creighton (1984) Proteins, W.H. Freeman and Company). In the case of substitutions, the amino acid replacing another amino acid usually has similar structural and/or chemical properties. Insertions and deletions are typically in the range of 1 to 5 amino acids, although depending upon the location of the insertion, more amino acids can be inserted or removed. The variations can be made using methods known in the art such as site-directed mutagenesis (Carter, et al. (1986) Nucl. Acids Res. 13:4331; Zoller et al. (1987) Nucl. Acids Res. 10:6487), cassette mutagenesis (Wells et al. (1985) Gene 34:315), restriction selection mutagenesis (Wells, et al. (1986) Philos. Trans. R. Soc. London SerA 317:415), and PCR mutagenesis (Sambrook et al., Molecular Cloning: A Laboratory Manual, 3rd edition, Cold Spring Harbor Press, N.Y., (2001)).

Two amino acid sequences are "substantially homologous" or "substantially similar" when greater than 80 %, preferably greater than 85 %, preferably greater than 90 % of the amino acids are identical, or greater than about 90 %, preferably grater than 95 %, are similar (functionally identical). Preferably, the similar or homologous sequences are identified by alignment using, for example, the GCG (Genetics Computer Group, Program Manual for the GCG Package, Version 7, Madison, Wisconsin) pileup program, or any of sequence comparison algorithms such as BLAST, FASTA, etc.

As used herein, the term "NP" denotes the measles virus nucleoprotein. The polypeptide and nucleotide sequences for measles virus nucleoprotein-are deposited in Genbank database under accession number X13480: The polypeptide sequence of NP is shown as SEQ ID NO:1.

As used herein, the term "T cells" includes lymphocytes which express phenotypic markers and rearrangements of the TCRb locus with or without rearrangements of the TCRa. Phenotypic markers include expression of CD4 and/or CD8 and CD3.

As used herein, "Th1 cells" refer to a subset of CD4+ T that produces IL-2, IFNg and lymphotoxin (LT also called TNFb). Th1 differentiation from naïve T cell is favoured by the presence of exogenous IFNg, IL-12 and IL-18. The expression of the IL-12R b2 subunit, T-bet and IL-18r may be considered as a marker for Th1 cells. Th1 play important roles in cellular immune functions such as delayed-type hypersensitivity or in the defence against intracellular organisms such as parasites.

As used herein, "Th2 cells" refer to another subset of cells that produce IL-4, IL-5, IL-6, IL-9, IL-10 and IL-13, but not IL-12 and IFNg. The essential cytokine for the development of Th2 cells is IL-4. Th2 cells generally enhance antibody production from B cells. The expression of GATA-3 may be considered as a marker for Th2 cells.

As used herein, a "specific regulatory immune response" is a cellular immune response corresponding to an induction of tolerance for the administrated epitope, which is also present in the atherosclerotic plaque, which can also dampen the immune response against any other epitope present in the micro-environment of the specific epitope by the so called bystander regulatory immune response and inhibit the local inflammatory response, through the release of IL-10 and of TGF-b.

As used herein, a "Treg response" corresponds to a response specific of "Treg cells" or "Tr cells" which refer to a distinct subset of T cells,. Tr cells exhibit a cytokine pattern distinct from Th1 and Th2 cells, through the release of immunosuppressive cytokines such as IL-10 and TGF-beta. Tr cells play a major role in induction of tolerance, largely by their ability to suppress responses mediated by other populations of T cells, especially Th1 cells. Tr cells include Tr1 cells and Th3 cells, which are characterized by the secretion of high amount of TGF-b. Tr1 cells secrete high levels of IL-10 and/or TGF-b, with or without IL-5 or IL-13, but little or no IL-2. There exists another subset of Tr cell that is antigen specific and can induce tolerance: CD4+CD25+ T cells. CD4+CD25+ T cells comprise 5-10% of the peripheral T cell pool and exhibit immunosuppressive abilities both in vitro and in vivo. Specifically, CD4+CD25+ T cells express the Foxp3 gene. This regulatory activity may depend on TGFb or cell-cell contact. Other Th1- or Th2-like cells may exert regulatory activity. As used herein, "Treg response" may be preceded or associated by the induction of tolerogenic antigen presenting cell response.

As used herein, the term "subject" denotes a Mammal, such as a rodent, a feline, a canine and a primate. The subject is an animal such as cow, pig, horse, chicken, cat, dog and most preferably a human.

By "purified" and "isolated" it is meant, when referring to a polypeptide (i.e. NP polypeptide) or a nucleotide sequence, that the indicated molecule is present in the substantial absence of other biological macromolecules of the same type. The term "purified" as used herein preferably means at least 75% by weight, more preferably at least 85% by weight, still preferably at least 95% by weight, and most preferably at least 98% by weight, of biological macromolecules of the same type are present. An "isolated" nucleic acid molecule which encodes a particular polypeptide refers to a nucleic acid molecule which is substantially free of other nucleic acid molecules that do not encode the subject polypeptide; however, the molecule may include some additional bases or moieties which do not deleteriously affect the basic characteristics of the composition.

### Measles virus nucleoprotein (NP) and measles virus (MV):

The core structure of measles virus (MV) is a pleomorphic ribonucleoprotein particle (RNP), consisting of the nonsegmented RNA genome of negative polarity tightly associated with viral polymerase complex and the nucleoprotein (NP) which encapsidates viral genomic RNA to form a helical nucleocapsid.

A profound suppression of immune functions during and after the acute infection is the major cause of more than one million cases of infant deaths associated with measles worldwide. Concomitant with the generation of an efficient MV specific immunity, immune responses towards other pathogens are strongly impaired and provide the basis for the establishment and sever course of opportunistic infections.

A marked lymphopaenia affecting mainly the T-cell population and a loss of delayed-type hypersensitivity reactions are characteristic of measles virus infections. One major hallmark of MV induced immunosuppression is the severe impairment of proliferative responses of lymphocytes to polyclonal and antigen-specific stimulation ex vivo. A cytokine imbalance as seen by predominant Th2 response and thereby suppression of cellular immunity has also been linked to immunosuppression (Griffin el al. 1995). Although MV was the first pathogen recognized to cause immunosuppression, only recently have the underlying mechanisms been partially unravelled.

One of the factors derived form the measles virus shown to be involved in the observed immunosuppression, is the measles virus nucleoprotein (NP), that possesses a broad immunosuppressive activity and is equally able to inhibit T cell dependent hypersensitivity, said inhibition being associated with the suppression of antigen specific T cell proliferation (Marie et al. 2001). The same authors concluded that the immunosuppressive effect of the measles virions was most probably composed of scored effects of free NP and released virion associated NP.

Thus the prior knowledge showed that measles virion, as well as several virions proteins, such as NP, despite being able to induce a useful immunity against measles virus itself, simultaneously induces a severe immunosuppression towards antigen heterologous to measles virus.

It has now been found by the inventors, that NP administration significantly reduces the development of new atherosclerotic lesions and markedly inhibits the progression of established plaques by promoting an anti-inflammatory T regulatory cell type 1-like response.

### Therapeutic methods and uses

The present invention provides for methods and compositions (such as pharmaceutical compositions) for treating and preventing atherosclerosis and /or atherosclerotic vascular diseases (AVD) which may affect the coronary arteries (causing ischaemic heart disease), the cerebral circulation (causing cerebrovascular disease), the aorta (producing aneurysms that are prone to thrombosis and rupture) and peripheral blood vessels, typically the legs (causing peripheral vascular disease and intermittent claudication). Ischaemic heart disease (IHD) includes angina (chest pain caused by insufficient blood supply to cardiac muscle) and myocardial infarction (death of cardiac muscle) and cerebrovascular disease includes stroke and transient ischaemic attacks.

In the context of the invention, the term "treating" or "treatment", as used herein, means reversing, alleviating, inhibiting the progress of, or preventing the disorder or condition to which such term applies, or one or more symptoms of such disorder or condition.

According to the invention, the term "patient" or "patient in need thereof", is intended for a human or non-human mammal affected or likely to be affected with atherosclerosis.

By a "therapeutically effective amount" of a polypeptide as above described is meant a sufficient amount of said polypeptide to treat atherosclerosis at a reasonable benefit/risk ratio applicable to any medical treatment. It will be understood, however, that the total daily usage of the compounds and compositions of the present invention will be decided by the attending physician within the scope of sound medical judgment. The specific therapeutically effective dose level for any particular patient will depend upon a variety of factors including the disorder being treated and the severity of the disorder; activity of the specific compound employed; the specific composition employed, the age, body weight, general health, sex and diet of the patient; the time of administration, route of administration, and rate of excretion of the specific compound employed; the duration of the treatment; drugs used in combination or coincidential with the specific polypeptide employed; and like factors well known in the medical arts. For example, it is well within the skill of the art to start doses of the compound at levels lower than those required to achieve the desired therapeutic effect and to gradually increase the dosage until the desired effect is achieved. However, the daily dosage of the products may be varied over a wide range from 0.01 to 1,000 mg per adult per day. Preferably, the compositions contain 0.01, 0.05, 0.1, 0.5, 1.0, 2.5, 5.0, 10.0, 15.0, 25.0, 50.0, 100, 250 and 500 mg of the active ingredient for the symptomatic adjustment of the dosage to the patient to be treated. A medicament typically contains from about 0.01 mg to about 500 mg of the active ingredient, preferably from 1 mg to about 100 mg of the active ingredient. An effective amount of the drug is ordinarily supplied at a dosage level from 0.0002 mg/kg to about 20 mg/kg of body weight per day, especially from about 0.001 mg/kg to 7 mg/kg of body weight per day.

In another embodiment the subject presents one of the following coronary disorders:
asymptomatic coronary artery coronary diseases with silent ischemia or without ischemia;
chronic ischemic disorders without myocardial necrosis, such as stable or effort angina pectoris;
acute ischemic disorders myocardial necrosis, such as unstable angina pectoris;
ischemic disorders with myocardial necrosis, such as ST segment elevation myocardial infarction or non-ST segment elevation myocardial
infarction.

Tissue ischemia occurs when the needs in oxygen exceed the delivery of oxygen to tissues. Myocardial ischemia can be diagnosed clinically (e.g. chest pain), biologically (e.g. increase in myeloperoxidase activity), metabolically, using scintigraphy, or by use of an electrocardiogram (typical modifications of the ST segment, upper or lower ST segment deviation, typical changes in T waves such as T wave inversion or steep asymmetric or high amplitude positives T waves). Silent ischemia is typically diagnosed using scintigraphy or a 24h electrocardiogram recording.

Chronic stable angina results from fixed stenoses in epicardial coronary arteries, which do not limit blood flow at rest, may become flow-limiting during periods of increased myocardial oxygen demand. Stenoses producing a 50% reduction in diameter, or a 70% reduction in cross-sectional area, are sufficient to impair the hyperaemic response that occurs with increasing cardiac work. Selective coronary arteriography is the gold standard for detecting such lesions, although it may underestimate the severity of coronary artery disease (CAD) in more diffusely diseased arteries. Angina pectoris is classically described as a retrosternal pressure that may radiate to the jaw, back, or left arm or shoulder. It may be associated with nausea, diaphoresis and a sense of impending doom. Stable angina is typically brought on by physical exertion, emotional stress, food and exposure to cold. Angina was originally studied in men, and the presentation of angina in women and the elderly tends to be less straightforward. Provocative stress testing with physical or pharmacological stress attempts to induce myocardial ischaemia in a controlled setting. The ischaemic zone may be detected as an electrically abnormal area on electrocardiagraphy (ECG), an area of impaired radionuclide intake with single photon emission computed tomography (SPECT), or as a wall motion abnormality on echocardiography. Patients with typical angina have a high pretest probability of CAD, but a negative test does not exclude the diagnosis.

Unstable angina is defined as new-onset angina, angina at rest, angina of increasing frequency and severity, or angina in the early post-Ml setting. The pathological correlate of unstable angina is rupture of an atherosclerotic plaque with formation of a flow-limiting, but nonocclusive, intracoronary platelet-rich thrombus. Vulnerable plaques are thought to have a thick lipid core with a thin fibrous cap and a preponderance of inflammatory cells. The ability to identify which plaques are unstable is limited. Intravascular ultrasonography intracoronary catheters, IRM and CT are methods under investigation. Unstable angina and non Q-wave myocardial infarction (NQWMI) are diagnosed by history, examination, ECG and laboratory studies. As noted above, rest pain is the hallmark of unstable angina. The ECG may show ST segment depression in the area of ischaemia, caused by abnormalities of polarization in the ischaemic tissue. The lack of pathologic Q waves on ECG signifies that the infarction is nontransmural. Prolonged ischaemia results in myocardial necrosis and release of the cardiac specific molecule troponin and creatine kinase (CK-MB) into the bloodstream. These markers of necrosis define the presence of MI and are usually evident only in retrospect. Therefore, unstable angina and NQWMI are usually grouped together for purposes of initial management.

Acute myocardial infarction is also known as Q-wave MI, transmural MI, or ST-elevation. It may occur suddenly or be preceded by unstable angina. The pathological correlate of acute MI is rupture and/or erosion of an atherosclerotic plaque with occlusive fibrin and platelet-rich intracoronary thrombus. Complete occlusion of the vessel results in transmural myocardial injury. The clinical presentation of patients with acute Ml is protean, ranging from mild chest pain to cardiogenic shock or sudden cardiac death. Physical examination may reveal only abnormal heart sounds or may demonstrate hypotension and pulmonary oedema. The ECG reveals ST-segment elevation in the area of ischaemia representing myocardial injury, and Q waves in areas of infarction. CK-MB and troponin levels are increased. Clinical features that predict a poor outcome include advanced patient age, tachycardia, low blood pressure, and the presence of pulmonary oedema and impaired tissue perfusion.

A further object of the invention relates to the use of a nucleic acid encoding for the polypeptides of the invention for the manufacture of a medicament intended for the treatment and/or the prevention of atherosclerosis.

A another object of the invention relates to a method for treating and/or preventing atherosclerosis comprising administering a subject in need thereof with a therapeutically effective amount of a nucleic acid encoding for the polypeptides of the invention.

Typically, the nucleic acid of the invention include DNA or RNA molecules, which may be inserted in any suitable vector, such as a plasmid, cosmid, episome, artificial chromosome, phage or a viral vector.

The terms "vector", "cloning vector" and "expression vector" mean the vehicle by which a DNA or RNA sequence (e.g. a foreign gene) can be introduced into a host cell, so as to transform the host and promote expression (e.g. transcription and translation) of the introduced sequence.

Such vectors may comprise regulatory elements, such as a promoter, enhancer, terminator and the like, to cause or direct expression of said polypeptide upon administration to a subject. The vectors may further comprise one or several origins of replication and/or selectable markers. The promoter region may be homologous or heterologous with respect to the coding sequence, and provide for ubiquitous, constitutive, regulated and/or tissue specific expression, in any appropriate host cell, including for in vivo use. Examples of promoters include bacterial promoters (T7, pTAC, Trp promoter, etc.), viral promoters (LTR, TK, CMV-IE, etc.), mammalian gene promoters (albumin, PGK, etc), and the like.

Examples of plasmids include replicating plasmids comprising an origin of replication, or integrative plasmids, such as for instance pUC, pcDNA, pBR, and the like. Examples of viral vector include adenoviral, retroviral, herpes virus and AAV vectors. Such recombinant viruses may be produced by techniques known in the art, such as by transfecting packaging cells or by transient transfection with helper plasmids or viruses. Typical examples of virus packaging cells include PA317 cells, PsiCRIP cells, GPenv+ cells, 293 cells, etc. Detailed protocols for producing such replication-defective recombinant viruses may be found for instance in WO 95/14785, WO 96/22378, US 5,882,877, US 6,013,516, US 4,861,719, US 5,278,056 and WO 94/19478.

### Function conservative variants of NP or fragment thereof:

A further object of the present invention encompasses the use of function-conservative variants of NP or fragments thereof.

According to the invention, function conservative variants of NP include polypeptides comprising an amino acid sequence homologous to the amino acid sequence as set forth in SEQ ID N0:1, and having anti-atherosclerosis activity.

Typically, according to the invention, function conservative variants of NP comprises a amino acid sequence having at least 70 or 80%, preferably at least 90% identity with SEQ ID NO:1, and more preferably at least 91 %, 92%, 93%, 94% 95%; 96%, 97%, 98%, or 99% identity with SEQ ID NO:1.

A further object of the invention encompasses the use of function conservative variants of the amino acid sequence as set forth in SEQ ID NO:2.
Typically the invention relates to the use of a polypeptide comprising an amino acid sequence having at least 80% identity with SEQ ID NO:2, said amino acid sequence having anti-atherosclerosis activity, for the manufacture of a medicament for the treatment and/or the prevention of atherosclerosis.
Typically, the amino acid sequence has at least 85%, preferably 90% and even more preferably 95% identity with SEQ ID NO: 2.

In addition to naturally occurring genetic variant of the above described amino acid sequences, the person skilled in the art will further appreciate that changes in amino acid sequences can be introduced by any technique well known in the art.

Modifications and changes may be made in the structure of the polypeptides of the present invention, and in the DNA sequences encoding them, and still obtain a functional molecule that encodes a polypeptide with desirable characteristics.

The amino acid changes may be achieved by changing codons in the DNA sequence, according to Table 1.

**Table 1**

| **Amino acids** | | | **Codons** |
|---|---|---|---|
| Alanine | Ala | A | GCA, GCC, GCG, GCU |
| Cysteine | Cys | C | UGC, UGU |
| Aspartic Acid | Asp | D | GAC, GAU |
| Glutamic acid | Glu | E | GAA, GAG |
| Phenylalanine | Phe | F | UUC, UUU |
| Glycine | Gly | G | GGA, GGC, GGG, GGU |
| Histidine | His | H | CAC, CAU |
| Isoleucine | Ile | I | AUA, AUC, AUU |
| Lysine | Lys | K | AAA, AAG |
| Leucine | Leu | L | UUA, UUG, CUA, CUC, CUG, CUU |
| Methionine | Met | M | AUG |
| Asparagine | Asn | N | AAC, AAU |
| Proline | Pro | P | CCA, CCC, CCG, CCU |
| Glutamine | Gln | Q | CAA, CAG |
| Arginine | Arg | R | AGA, AGG, CGA, CGC, CGG, CGU |
| Serine | Ser | S | AGC, AGU, UCA, UCC, UCG, UCU |
| Threonine | Thr | T | ACA, ACC, ACG, ACU |
| Valine | Val | V | GUA, GUC, GUG, GUU |
| Tryptophan | Trp | W | UGG |
| Tyrosine | Tyr | Y | UAU |

For example, certain amino acids may be substituted for other amino acids in a protein structure without appreciable loss of anti-angiogenic capability. Since it is the interactive capacity and nature of a protein that defines that protein's biological functional activity, certain amino acid substitutions can be made in a protein sequence, and, of course, in its DNA encoding sequence, and nevertheless obtain a protein with like properties. It is thus contemplated that various changes may be made in the polypeptide sequences of the invention, or corresponding DNA sequences which encode said polypeptides, without appreciable loss of their biological activity.

In making the changes in the amino sequences of polypeptide, the hydropathic index of amino acids may be considered. The importance of the hydropathic amino acid index in conferring interactive biologic function on a protein is generally understood in the art. It is accepted that the relative hydropathic character of the amino acid contributes to the secondary structure of the resultant protein, which in turn defines the interaction of the protein with other molecules, for example, enzymes, substrates, receptors, DNA, antibodies, antigens, and the like. Each amino acid has been assigned a hydropathic index on the basis of their hydrophobicity and charge characteristics these are: isoleucine (+4.5); valine (+4.2); leucine (+3. 8) ; phenylalanine (+2.8); cysteine/cystine (+2.5); methionine (+1.9); alanine (+1.8); glycine (-0.4); threonine (-0.7); serine (-0.8); tryptophane (-0.9); tyrosine (-1.3); proline (-1.6); histidine (-3.2); glutamate (-3.5); glutamine (-3.5); aspartate (<RTI (?) 3.5); asparagine (-3.5); lysine (-3.9); and arginine (-4.5).

It is known in the art that certain amino acids may be substituted by other amino acids having a similar hydropathic index or score and still result in a protein with similar biological activity, i.e. still obtain a biological functionally equivalent protein.

As outlined above, amino acid substitutions are generally therefore based on the relative similarity of the amino acid side-chain substituents, for example, their hydrophobicity, hydrophilicity, charge, size, and the like. Exemplary substitutions which take various of the foregoing characteristics into consideration are well known to those of skill in the art and include: arginine and lysine; glutamate and aspartate; serine and threonine; glutamine and asparagine; and valine, leucine and isoleucine.

An further object of the invention encompasses the use of a nucleic acid encoding for any function conservative variants as above described.

### Methods for producing polypeptides of the inventions :

The polypeptides of the invention may be produced by any technique known per se in the art, such as, without limitation, any chemical, biological, genetic or enzymatic technique, either alone or in combination.

Knowing the amino acid sequence of the desired sequence, one skilled in the art can readily produce said polypeptides, by standard techniques for production of polypeptides. For instance, they can be synthesized using well-known solid phase method, preferably using a commercially available peptide synthesis apparatus (such as that made by Applied Biosystems, Foster City, California) and following the manufacturer's instructions.

Alternatively, the polypeptides of the invention can be synthesized by recombinant DNA techniques as is now well-known in the art. For example, these fragments can be obtained as DNA expression products after incorporation of DNA sequences encoding the desired (poly)peptide into expression vectors and introduction of such vectors into suitable eukaryotic or prokaryotic hosts that will express the desired polypeptide, from which they can be later isolated using well-known techniques.

For example, nucleic acids encoding for NP polypeptides of the invention may be used to produce a recombinant polypeptide of the invention in a suitable expression system.

The term "expression system" means a host cell and compatible vector under suitable conditions, e.g. for the expression of a protein coded for by foreign DNA carried by the vector and introduced to the host cell.

Common expression systems include E. coli host cells and plasmid vectors, insect host cells and *Baculovirus vectors,* and mammalian host cells and vectors. Other examples of host cells include, without limitation, prokaryotic cells (such as bacteria) and eukaryotic cells (such as yeast cells, mammalian cells, insect cells, plant cells, etc.). Specific examples include therefore *E.coli, Baculovirus,* mammalian cell lines (e.g., Vero cells, CHO cells, 3T3 cells, COS cells, etc.) as well as primary or established mammalian cell cultures.

Polypeptides of the invention can be use in an isolated (e.g., purified) form or contained in a vector, such as a membrane or lipid vesicle (e.g. a liposome) or nanoparticles.

### Pharmaceutical compositions

The polypeptides of the invention or nucleic acid encoding for them may be combined with pharmaceutically acceptable excipients, and optionally sustained-release matrices, such as biodegradable polymers, to form therapeutic compositions.

"Pharmaceutically" or "pharmaceutically acceptable" refers to molecular entities and compositions that do not produce an adverse, allergic or other untoward reaction when administered to a mammal, especially a human, as appropriate. A pharmaceutically acceptable carrier or excipient refers to a non-toxic solid, semi-solid or liquid filler, diluent, encapsulating material or formulation auxiliary of any type.

In the pharmaceutical compositions of the present invention for oral, sublingual, subcutaneous, intramuscular, intravenous, transdermal, local or rectal administration, the active principle, alone or in combination with another active principle, can be administered in a unit administration form, as a mixture with conventional pharmaceutical supports, to animals and human beings. Suitable unit administration forms comprise oral-route forms such as tablets, gel capsules, powders, granules and oral suspensions or solutions, sublingual and buccal administration forms, aerosols, implants, subcutaneous, transdermal, topical, intraperitoneal, intramuscular, intravenous, subdermal, transdermal, intrathecal and intranasal administration forms and rectal administration forms.

Preferably, the pharmaceutical compositions contain vehicles which are pharmaceutically acceptable for a formulation capable of being injected. These may be in particular isotonic, sterile, saline solutions (monosodium or disodium phosphate, sodium, potassium, calcium or magnesium chloride and the like or mixtures of such salts), or dry, especially freeze-dried compositions which upon addition, depending on the case, of sterilized water or physiological saline, permit the constitution of injectable solutions.

The pharmaceutical forms suitable for injectable use include sterile aqueous solutions or dispersions; formulations including sesame oil, peanut oil or aqueous propylene glycol ; and sterile powders for the extemporaneous preparation of sterile injectable solutions or dispersions. In all cases, the form must be sterile and must be fluid to the extent that easy syringability exists. It must be stable under the conditions of manufacture and storage and must be preserved against the contaminating action of microorganisms, such as bacteria and fungi.

Solutions comprising compounds of the invention as free base or pharmacologically acceptable salts can be prepared in water suitably mixed with a surfactant, such as hydroxypropylcellulose. Dispersions can also be prepared in glycerol, liquid polyethylene glycols, and mixtures thereof and in oils. Under ordinary conditions of storage and use, these preparations contain a preservative to prevent the growth of microorganisms.

Polypeptides of the invention can be formulated into a composition in a neutral or salt form. Pharmaceutically acceptable salts include the acid addition salts (formed with the free amino groups of the protein) and which are formed with inorganic acids such as, for example, hydrochloric or phosphoric acids, or such organic acids as acetic, oxalic, tartaric, mandelic, and the like. Salts formed with the free carboxyl groups can also be derived from inorganic bases such as, for example, sodium, potassium, ammonium, calcium, or ferric hydroxides, and such organic bases as isopropylamine, trimethylamine, histidine, procaine and the like.

The carrier can also be a solvent or dispersion medium containing, for example, water, ethanol, polyol (for example, glycerol, propylene glycol, and liquid polyethylene glycol, and the like), suitable mixtures thereof, and vegetables oils. The proper fluidity can be maintained, for example, by the use of a coating, such as lecithin, by the maintenance of the required particle size in the case of dispersion and by the use of surfactants. The prevention of the action of microorganisms can be brought about by various antibacterial and antifungal agents, for example, parabens, chlorobutanol, phenol, sorbic acid, thimerosal, and the like. In many cases, it will be preferable to include isotonic agents, for example, sugars or sodium chloride. Prolonged absorption of the injectable compositions can be brought about by the use in the compositions of agents delaying absorption, for example, aluminium monostearate and gelatin.

Sterile injectable solutions are prepared by incorporating the active polypeptides in the required amount in the appropriate solvent with various of the other ingredients enumerated above, as required, followed by filtered sterilization. Generally, dispersions are prepared by incorporating the various sterilized active ingredients into a sterile vehicle which contains the basic dispersion medium and the required other ingredients from those enumerated above. In the case of sterile powders for the preparation of sterile injectable solutions, the preferred methods of preparation are vacuum-drying and freeze-drying techniques which yield a powder of the active ingredient plus any additional desired ingredient from a previously sterile-filtered solution thereof.

Upon formulation, solutions will be administered in a manner compatible with the dosage formulation and in such amount as is therapeutically effective. The formulations are easily administered in a variety of dosage forms, such as the type of injectable solutions described above, but drug release capsules and the like can also be employed.

For parenteral administration in an aqueous solution, for example, the solution should be suitably buffered if necessary and the liquid diluent first rendered isotonic with sufficient saline or glucose. These particular aqueous solutions are especially suitable for intravenous, intramuscular, subcutaneous and intraperitoneal administration. In this connection, sterile aqueous media which can be employed will be known to those of skill in the art in light of the present disclosure. For example, one dosage could be dissolved in 1 ml of isotonic NaCl solution or injected at the proposed site of infusion. Some variation in dosage will necessarily occur depending on the condition of the subject being treated. The person responsible for administration will, in any event, determine the appropriate dose for the individual subject.

The compounds of the invention may be formulated within a therapeutic mixture to comprise about 0.0001 to 1.0 milligrams, or about 0.001 to 0.1 milligrams, or about 0.1 to 1.0 or even about 10 milligrams per dose or so. Multiple doses can also be administered.

In addition to the compounds of the invention formulated for parenteral administration, such as intravenous or intramuscular injection, other pharmaceutically acceptable forms include, e.g. tablets or other solids for oral administration; liposomal formulations; time release capsules ; and any other form currently used, such as nanoparticles.

The invention will further be illustrated in view of the following figures and examples.

### FIGURES:

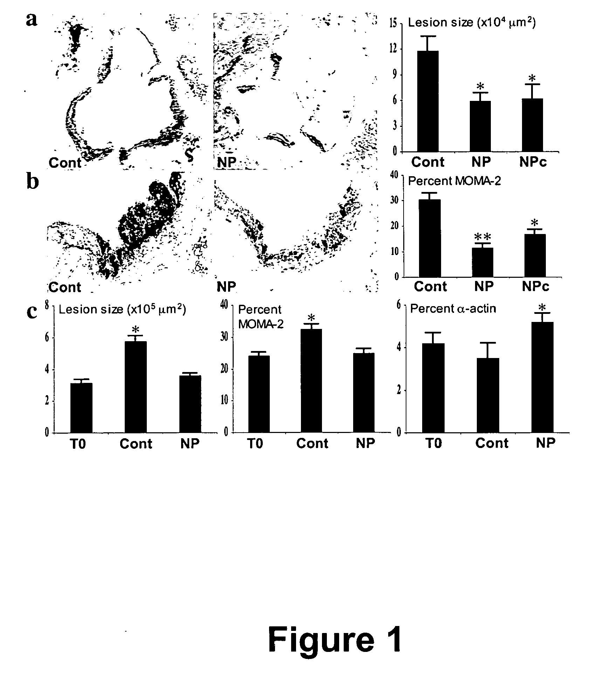

### Figure 1. Treatment with measles virus nucleoprotein reduces the formation of early atherosclerotic lesions and inhibits the progression of established plaques.

**(a)** Representative photomicrographs of Oil red O staining (left and middle panels) and quantitative analysis (right panel) of atherosclerotic lesion size in the aortic sinus of male *Apoe*^{*-*/*-*} mice injected intraperitoneally at the age of 12 weeks with either phosphate-buffered saline (Cont; n=8), 20 µg of measles virus nucleoprotein (NP; n=6), or 30 µg of its the C-terminal part (NPc; n=6), every other week. Mice were sacrified following the seventh injection. Values represent mean values ± s.e.m.; single asterisk, P < 0.05 vs control.
**(b)** Representative photomicrographs of macrophage staining with MOMA-2 (left and middle panels) and quantitative analysis (right panel) of the percentage of lesion area occupied by macrophages in control, NP-treated or NPc-treated Apoe-/mice. Values represent mean values ± s.e.m.; single asterisk, P < 0.05 vs control; double asterisk, P < 0.01 vs control.
**(c)** Quantitative analysis of atherosclerotic lesion size (left panel) in the aortic sinus of male Apoe-/- mice sacrified at the age of 30 weeks (T0; n=5) or sacrified at the age of 42 weeks after intraperitoneal injections of either phosphate-buffered saline (Cont; n=9) or 20 µg of NP (n=9) every other week for 12 weeks. Values represent mean values ± s.e.m.; single asterisk, P < 0.001 vs TO and NP. Middle panel shows quantitative analysis of MOMA-2 staining (macrophage content) in aortic sinus lesions. Values represent mean values ± s.e.m.; single asterisk, P < 0.05 vs T0 and NP. Right panel shows quantitative analysis of a-actin staining (smooth muscle cell content) in aortic sinus lesions. Values represent mean values ± s.e.m.; single asterisk, P < 0.05 vs Cont.

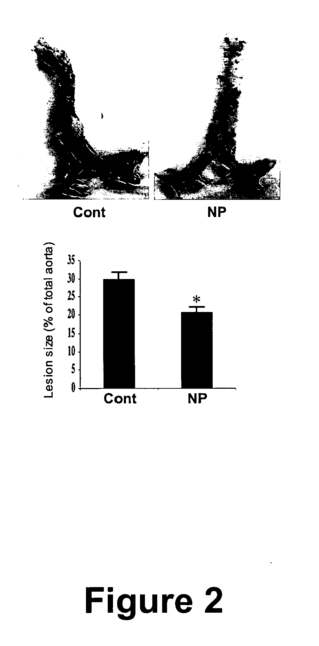

### Figure 2. Treatment with measles virus nucleoprotein reduces the progression of established atherosclerotic lesions.

Representative photomicrographs of Oil red O staining and quantitative analysis of atherosclerotic lesion size in the thoracic aorta of 30-week old male Apoe-/- mice injected intraperitoneally with either phosphate-buffered saline (Cont; n=4), or 20 µg of measles virus nucleoprotein (NP; n=9), every other week. Mice were sacrified at 42 weeks of age 24 to 48 h following the seventh injection. Values represent mean values ± s.e.m.; single asterisk, P < 0.05 vs control.

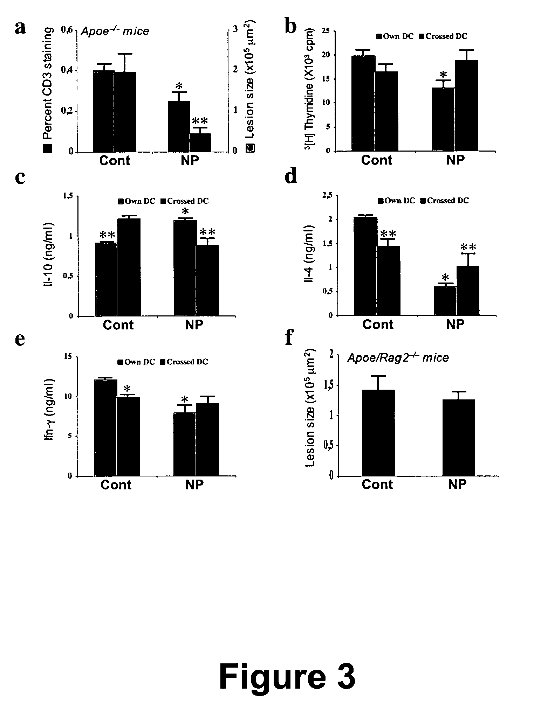

### Figure 3. Treatment with measles virus nucleoprotein induces a regulatory immune response.

**(a)** Quantitative analysis of lesion size and T lymphocyte infiltration (CD3-positive staining) in aortic sinus atherosclerotic lesions of male *Apoe*^{*-*/*-*} mice injected intraperitoneally at the age of 12 weeks with either phosphate-buffered saline (Cont; n=7) or 20 µg of measles virus nucleoprotein (NP; n=6) every other week for 12 weeks. Values represent mean values ± s.e.m.; single asterisk, P < 0.05; double asterisks, P < 0.01.
**(b)** Proliferation of purified CD4+ T cells (10⁵/well) in the presence of purified CD11 c+ dendritic cells (2x10⁴/well). Cells were recovered from spleens and lymph nodes of 12-week old male Apoe-/- mice injected intraperitoneally at the age of 12 weeks with either phosphate-buffered saline (Cont; n=3) or 20 µg of measles virus nucleoprotein (NP; n=3) every other week for 6 weeks and sacrified 24 hours following the fourth injection. Cells were stimulated in vitro with soluble anti-CD3 antibody (1 µg/ml) for 96 hours. Tritiated thymidine was added for the last 18 hours of culture. Values represent mean values ± s.e.m. of triplicates; single asterisk, P < 0.05 vs Cont/own DC.
**(c-e)** Cytokine production in the supernatants of purified CD4+ T cells (10⁵/well) stimulated for 48 hours in wells coated with anti-CD3 antibody (5 µg/ml) in the presence of purified CD11 c+ dendritic cells (2x10⁴/well). Cells were isolated from the animals described above. Values represent mean values ± s.e.m. of triplicates. CD4+ T cells of mice treated with NP showed enhanced II-10 (single asterisk, P < 0.05 vs Cont/own DC and NP/crossed DC; double asterisk, P < 0.05 vs Cont/crossed DC) but reduced II-4 (single asterisk, P < 0.05 vs Cont/own DC, Cont/crossed DC and NP/crossed DC; double asterisk, P < 0.05 vs Cont/own DC and NP/own DC) and Ifn-g production (single asterisk, P < 0.05 vs Cont/own DC). The presence of dendritic cells recovered from mice treated with NP appears to favour an anti-inflammatory Tr1-like cytokine profile.
**(f)** Quantitative analysis of atherosclerotic lesion size in the aortic sinus of Apoe/Rag2-/- mice fed a cholate-free high fat diet and injected intraperitoneally the age of 8 weeks with either phosphate-buffered saline (Cont; n=6) or 20 µg of measles virus nucleoprotein (NP; n=8) every other week for 6 weeks. Values represent mean values ± s.e.m.; NP treatment did not alter lesion size in mice with lymphocyte deficiency.

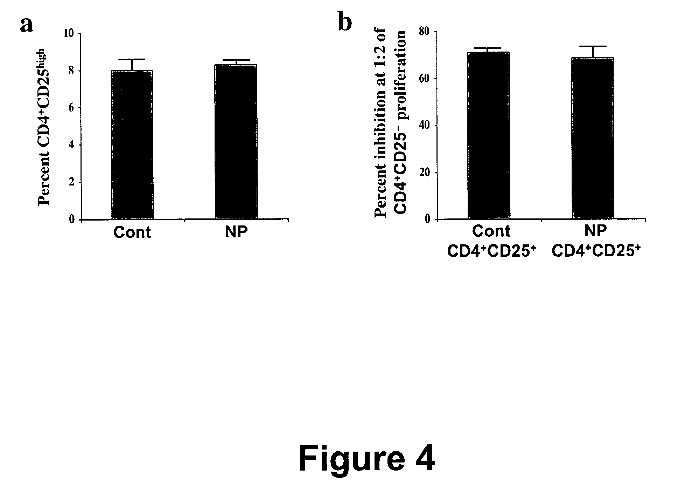

### Figure 4. Assessment of natural regulatory T cell function.

Panel (a), shows similar levels of CD4+CD25+ in the spleens of control and NP-treated Apoe-/- mice using flow cytometry. Values are mean ± s.e.m. of n=3 mice per group. Panel (b), shows suppression of proliferation of CD4+CD25- effector cells by CD4+CD25+ cells from control or NP-treated mice. Proliferation was assessed by 3[H] thymidine incorporation after stimulation with soluble anti-CD3 in the presence of purified CD11 c+ dendritic cells. Results are expressed as percentage inhibition of CD4+CD25- effector cells. Cells were purified and pooled from 3 mice in each group. Values are mean ± s.e.m. of triplicate measurements

### EXAMPLE:

### Materials and methods

**Production of recombinant measles virus nucleoprotein.** We produced and purified recombinant measles virus (MV) nucleoprotein (525 aa) and its C-terminal part NPc (125aa) using recombinant baculovirus as described previously (Kerdiles et al, Viral Immunol 2006). Briefly, we inserted MV-NP cDNA or MV-NPc cDNA of the Hallé strain into pAcHLT baculovirus transfer vectors containing a 6xHis tag (BD baculogold 6xHis Expression and purification kit, BD Biosciences). Recombinants baculoviruses were then generated in Sf9 insect cell line (ATCC CRL-1711) by homologous recombination with linearized Baculogold, according to manufacturer's instructions (BD baculogold 6xHis Expression and purification kit, BD Biosciences). For recombinant protein production confluent monolayers of High Five insect cells (Invitrogen) were infected with the corresponding virus at a multiplicity of 1 PFU/cell and infected cells were harvested 4 days post infection, pelleted and stored at -80°C until protein purification. Nucleocapsid formation in infected insect cells allowed the purification of recombinant NP on discontinuous CsCl gradients, whereas NPc was purified by affinity chromatography on Ni-NTA Agarose column according to manufacturer's instructions (BD baculogold 6xHis Expression and purification kit, BD Biosciences). Once purified, each batch of NP or NPc were aliquoted in PBS, analyzed by 10% SDS-Page and Western blot and stored at -80°C.

**Animals.** We used several sets of male Apoe-/- mice of 12 or 30 weeks of age (Charles River, Orléans, France). Mice were fed a chow diet and treated, as indicated, with either phosphate-buffered saline, purified recombinant NP or purified recombinant NPc. We also used Apoe/Rag2-/- mice at 8 weeks of age. These mice were fed a cholate-free, high fat diet (1.25% cholesterol, 15% fat) in order to induce substantial lesion formation, similar to those observed in Apoe-/- mice. These mice also received, as indicated, injections of phosphate-buffered saline or purified recombinant NP. Experiments were conducted according to the French veterinary guidelines and those formulated by the European community for experimental animal use (L358-86/609EEC).

**Extent and composition of atherosclerotic lesions.** We measured plasma cholesterol using a commercial cholesterol kit (Biomerieux). The heart of mice was removed and we obtained successive 10-µm transversal sections of aortic sinus. Lipids and collagen were detected using Oil red O and Sirius red stainings, respectively. We studied the presence of macrophages, T lymphocytes, and smooth muscle cells using specific antibodies, as previously described (Mallat et al. 2003). At least 4 sections per mouse were examined for each immunostaining, and appropriate negative controls were used. Thoracic aortas were dissected and stained with Oil red O. We performed morphometric studies using Histolab software (Microvisions) (Mallat et al. 2003).

**Cell recovery and purification.** We performed negative selection of CD4+ cells by use of a cocktail of antibody-coated magnetic beads from Miltenyi Biotech (DX5, ter 119, CD8a, CD11b, and CD45R-specific antibodies), and purified dendritic cells using CD11c-coated magnetic beads according to manufacturer's instructions (Miltenyi Biotech). In some experiments, we purified regulatory and effector T cells with biotin-conjugated anti-CD25 mAb (7D4, PharMingen), streptavidin microbeads (Miltenyi Biotec), followed by 2 consecutive magnetic cell separations using LS columns (Miltenyi Biotec), giving 80% CD4+CD25+ cells. The CD25- cells, which did not bind to the beads, were harvested from the flow through and contained less than 0.3% of CD4+CD25+ T-cells.

**Cell culture, proliferation and cytokine assays.** Cells were cultured in RPMI 1640 supplemented with Glutamax, 10% FCS, 0.02 mM 2β-mercaptoethanol and antibiotics. To assess in vitro proliferation of purified CD4+ T cells, we cultured them in flat-bottomed 96-well microplates (10⁵ cells/well; total volume 200 µl/well) in the presence of antigen-presenting cells (2x10⁴ cells/well) purified on CD11c-coated magnetic beads (Miltenyi Biotech) and stimulated the cells with purified soluble CD3-specific antibody (1 µg/ml, Pharmingen). Cells were cultured at 37°C for 96 h and pulsed with 1 pCi of 3[H] thymidine (Amersham) for the last 18 h of culture. Thymidine incorporation was assessed using a TopCount NXT scintillation counter (Perkin Elmer). To assess in vitro suppressive potential of natural regulatory T cells, we cultured CD4+CD25+ and CD4+CD25- cells alone or in co-culture (at a 1:1 ratio) in flat-bottomed 96-well microplates (0.5x10⁵ cells/well; total volume 200 µl/well). We stimulated cells with purified soluble CD3-specific antibody (1 µg/ml, Pharmingen) in the presence of CD11 c+ cells. Cells were cultured at 37°C for 72 h and pulsed with 1 µCi of 3[H] thymidine (Amersham) for the last 18 h of culture. For cytokine measurements, we cultured purified CD4+ T cells at 1x10⁵ cells/well for 48 h in anti-CD3-coated microplates (5 µg/ml). II-4, II-10 and Ifn-γ productions in the supernatant were measured using specific ELISAs (R&D Systems).

**Flow cytometry.** We labeled cells with allophycocyanin-conjugated antibody to CD4 (RM4-5, PharMingen), and phycoerythrin-conjugated CD25-specific antibody (PC61, PharMingen) and then analyzed the cells by flow cytometry.

**Statistical analysis.** Values are expressed as means ± s.e.m. Differences between values were examined using Mann-Whitney test and were considered significant at P<0.05.

### Results :

### Effects of NP administration on the development of early atherosclerotic lesions :

Male apolipoprotein E deficient (Apoe-/-) mice have very small lipid lesions in the aortic sinus at the age of 12 weeks. We treated male Apoe-/- mice with intraperitoneal injections of 20 µg of purified recombinant NP every other week. Mice were sacrified 24 to 48 hours following the seventh NP injection. This dose was chosen based on previous studies from one of our groups showing that the immunosuppressive effects of NP reached a threshold at 12 µg, and were maximal with 25 µg of NP (Marie et al., 2001). Control male Apoe-/- mice received repeated injections of phosphate-buffered saline. Weights (29.5 ± 1.5 vs 29.5 ± 0.5 g in control and NP groups, respectively, P=0.95) and serum total cholesterol levels (4.8 ± 0.3 vs 4.3 ± 0.7 g/l in control and NP groups, respectively, P=0.61) were similar between control and NP-treated mice. We observed an important and significant 50% reduction (P<0.05) in the size of atherosclerotic lesions in mice treated with NP compared with controls (Fig. 1a). Lesions of mice treated with NP also showed marked 62% reduction in the accumulation of macrophages (P<0.05), the main component of inflammatory lipid lesions, indicating reduced plaque inflammation (Fig. 1 b).

In addition, treatment of male Apoe-/- mice with the C-terminal part of NP, NPc, known to display similar immunosuppressive effects as NP (Marie et al. 2001), also led to a significant 48% reduction in lesion size (P<0.05; Fig.1 a) and 45% reduction in macrophage infiltration (P<0.05; Fig.1 b), despite similar cholesterol levels (4.9 ± 0.3 g/I, P=0.61).

In order to further confirm our findings, we repeated the experiment using another set of male Apoe-/- mice and obtained similar results. Again, treatment with NP led to significant reduction in lesion size (123 628 ± 24 225 µm² vs 200 894 ± 16 782 µm² in NP-treated, n=7 and control-treated mice, n=6, respectively, P<0.05)(Fig.2a).

### Effects of NP treatment on the progression and composition of established atherosclerotic plaques in mice :

In the vast majority of cases, individuals in need of an anti-atherosclerosis therapy already have established atherosclerotic plaques. An effective therapy in this population should induce plaque stabilization (reduction in macrophages and enhancement of smooth muscle cell content) (Libby et al., 2002) and/or limit plaque progression.

Thus, we examined the effects of NP treatment on the progression and composition of established atherosclerotic plaques in mice. Male Apoe-/- mice have established lipid lesions in the aortic sinus at the age of 30 weeks (Fig. 1 c). We treated 30-week old male Apoe-/- mice with 20 µg of purified recombinant NP intraperitoneally every other week and mice were sacrified 24 to 48 hours following the seventh NP injection. Control male Apoe-/- mice received repeated injections of phosphate-buffered saline. Weights (35.3 ± 1.0 vs 36.9 ± 1.2 g in control and NP groups, respectively) and serum total cholesterol levels (6.1 ± 0.6 vs 6.7 ± 0.7 g/l in control and NP groups, respectively) were similar between control and NP-treated mice. We observed an important and significant 38% reduction (P<0.001) in the size of atherosclerotic lesions in mice treated with NP compared with controls (Fig. 1 c). Interestingly, NP treatment of Apoe-/- mice at the age of 30 weeks almost blocked further age-related plaque progression. During the 12-week period of treatment, mice treated with NP showed 6-fold reduction in lesion progression compared with control mice (mean increase in lesion size: 14.8 ± 4.6% in NP-treated mice vs 84.2 ± 11.5% in control mice, P<0.001) (Fig. 1c). NP treatment also blocked further macrophage accumulation within the lesions (P<0.05 vs control mice), and significantly enhanced smooth muscle cell content (P<0.05 vs control), suggesting a switch toward a stable plaque phenotype (Fig. 1 c). We also examined lesion progression in the thoracic aorta, another atherosclerosis-prone site. Before treatment, lesions occupied 14.9 ± 2.4% of total aortic area. Twelve weeks later, we found a marked inhibition of plaque progression in NP-treated mice (20.7 ± 1.8%, P=0.13 vs before treatment) compared with control-treated mice (29.8 ± 2.1 %, P=0.014 vs before treatment and P=0.02 vs NP group) (Figure 2). Similar effects were obtained with NPc treatment, leading to 58.7% reduction in lesion progression at the level of the aortic sinus (mean increase in lesion size: 34.8% in NPc-treated mice vs 84.2% in control mice, P<0.01).

Thus, NP treatment reduces the development of early lipid lesions and significantly inhibits the progression of established plaques.

### Study of mechanisms responsible for NP protective effects in atherosclerosis :

We next examined potential mechanisms responsible for NP protective effects in atherosclerosis. An important feature of the lipid lesions of Apoe-/- mice treated with NP was a very important reduction in the accumulation of CD3-positive T lymphocytes (Fig. 3a).

Thus, we examined the effects of NP treatment (6 weeks) on the proliferation and cytokine production of purified CD4+ T cells in the presence of dendritic cells. We observed a significant reduction in CD4+ T cell proliferation in vitro in response to CD3 stimulation when cells were recovered from NP-treated mice compared with control-treated mice (Fig. 3b). Incubation of dendritic cells from control mice with CD4+ T cells of NP-treated mice rescued T cell proliferation, whereas incubation of dendritic cells from NP-treated mice with CD4+ T cells of control mice inhibited T cell proliferation (Fig. 3b), suggesting a dendritic cell-dependent effect of NP on T cell proliferation. The reduction in T cell proliferation was not associated with a better natural regulatory T cell function, since we found similar levels of CD4+CD25+ cells, Foxp3 expression and similar in vitro suppressive potential of CD4+CD25+ cells in NP-treated and control mice (Figure 4). Besides CD4⁺CD25⁺ naturally occurring regulatory T cells, other inducible CD4⁺ cells with potent immuno-regulatory properties have been described. In particular T regulatory cells type 1 (Tr1) are CD4+ T lymphocytes that are defined by their production of II-10 and suppression of T helper cells (Groux et al., 1997; Kemper et al., 2003). Therefore, we assessed cytokine production in T cell supernatants after CD3 stimulation in the presence of dendritic cells. Interestingly, we found a dendritic cell-dependent increase in T cell production of II-10 (Fig. 3c), and a significant reduction in both II-4 (Fig. 3d), and lfn-g (Fig. 3e), suggesting the induction of a Tr1-like phenotype suppressing both Th1 and Th2 responses.

In order to examine whether the induction of a Tr1-like immune response by NP was required for NP protective effects in atherosclerosis, we repeated NP injections in Apoe/Rag2-/- mice, which are deficient in T cells. Interestingly, we found no effect on lesion size in Apoe/Rag2-/- mice treated with NP compared with control Apoe/Rag2-/- mice (Fig. 3f), suggesting that the induction of a regulatory T cell immune response after NP treatment is necessary for its anti-atherosclerotic effect.

### Conclusion :

In conclusion, we show that repetitive administration of measles virus nucleoprotein to mice susceptible to atherosclerosis induces a regulatory immune response as revealed by a reduction in T lymphocyte proliferation, a reduction in Ifn-γ and II-4, and an increase in II-10 production, leading to a limitation of lesion development and progression. Our findings identify a novel mechanism of immune modulation by measles virus nucleoprotein through the promotion of a Tr1-like response and indicate that measles virus nucleoprotein (NP), variants or fragments thereof may be used for the treatment and/or the prevention of atherosclerosis.

### References :

Ait-Oufella H, Salomon BL, Potteaux S, Robertson AK, Gourdy P, Zoll J, Merval R, Esposito B, Cohen JL, Fisson S, Flavell RA, Hansson GK, Klatzmann D, Tedgui A, Mallat Z. Natural regulatory T cells control the development of atherosclerosis in mice. Nat Med. 2006 Feb;12(2):178-80.

Binder CJ, Chang MK, Shaw PX, Miller YI, Hartvigsen K, Dewan A, Witztum JL. Innate and acquired immunity in atherogenesis. Nat Med. 2002 Nov;8(11):1218-26.

Davenport P, Tipping PG. The role of interleukin-4 and interleukin-12 in the progression of atherosclerosis in apolipoprotein E-deficient mice. Am J Pathol. 2003 Sep;163(3):1117-25.

Griffin DE, Ward BJ, Esolen LM. Pathogenesis of measles virus infection: an hypothesis for altered immune responses. J Infect Dis. 1994 Nov;170 Suppl 1:S24-31.

Groux H, O'Garra A, Bigler M, Rouleau M, Antonenko S, de Vries JE, Roncarolo MG. A CD4+ T-cell subset inhibits antigen-specific T-cell responses and prevents colitis. Nature. 1997 Oct 16;389(6652):737-42.

Gupta S, Pablo AM, Jiang X, Wang N, Tall AR, Schindler C. IFN-gamma potentiates atherosclerosis in ApoE knock-out mice. J Clin Invest. 1997 Jun 1;99(11):2752-61.

Hansson GK, Libby P. The immune response in atherosclerosis: a double-edged sword. Nat Rev Immunol. 2006 Jul;6(7):508-19.

Kemper C, Chan AC, Green JM, Brett KA, Murphy KM, Atkinson JP. Activation of human CD4+ cells with CD3 and CD46 induces a T-regulatory cell 1 phenotype. Nature. 2003 Jan 23;421 (6921):388-92.

Kerdiles YM, Cherif B, Marie JC, Tremillon N, Blanquier B, Libeau G, Diallo A, Wild TF, Villiers MB, Horvat B. Immunomodulatory properties of morbillivirus nucleoproteins. Viral immunol. 2006 Summer; 19(2):324-34.

Mallat Z, Gojova A, Brun V, Esposito B, Fournier N, Cottrez F, Tedgui A, Groux H. Induction of a regulatory T cell type 1 response reduces the development of atherosclerosis in apolipoprotein E-knockout mice. Circulation. 2003 Sep 9;108(10):1232-7.

Maloy KJ, Powrie F. Regulatory T cells in the control of immune pathology. Nat Immunol. 2001 Sep;2(9):816-22.

Marie JC, Kehren J, Trescol-Biemont MC, Evlashev A, Valentin H, Walzer T, Tedone R, Loveland B, Nicolas JF, Rabourdin-Combe C, Horvat B. Mechanism of measles virus-induced suppression of inflammatory immune responses. Immunity. 2001 Jan;14(1):69-79.

Robertson AK, Rudling M, Zhou X, Gorelik L, Flavell RA, Hansson GK. Disruption of TGF-beta signaling in T cells accelerates atherosclerosis. J Clin Invest. 2003 Nov;112(9):1342-50.

Sakaguchi S Naturally arising Foxp3-expressing CD25+CD4+ regulatory T cells in immunological tolerance to self and non-self. Nat Immunol. 2005 Apr;6(4):345-52.

Tedgui A, Mallat Z. Cytokines in atherosclerosis: pathogenic and regulatory pathways. Physiol Rev. 2006 Apr;86(2):515-81.

Whitman SC, Ravisankar P, Elam H, Daugherty A. Exogenous interferon-gamma enhances atherosclerosis in apolipoprotein E-/- mice. Am J Pathol. 2000 Dec;157(6):1819-24.

## Claims

1. Use of a polypeptide comprising the amino acid sequence as set forth in SEQ ID NO:2 for the manufacture of a medicament for the treatment and/or the prevention of atherosclerosis.

2. Use of a polypeptide comprising an amino acid sequence having at least 80% identity with SEQ ID NO:2, said amino acid sequence having anti-atherosclerosis activity, for the manufacture of a medicament for the treatment and/or the prevention of atherosclerosis.

3. The use according to claim 2, wherein the amino acid sequence has at least 90% identity with SEQ ID NO:2.

4. Use of a polypeptide comprising a fragment of the amino acid sequence of any one of claims 1 to 3, said fragment having anti-atherosclerosis activity, for the manufacture of a medicament for the treatment and/or the prevention of atherosclerosis.

5. Use according to any one of claims 1 to 4 wherein said polypeptide comprises the amino acid sequence as set forth in SEQ ID N0:1 or an amino acid sequence having at least 70% identity with the amino acid sequence as set forth in SEQ ID N0:1.

6. Use of a nucleic acid encoding a polypeptide according to any one of claims 1 to 5, for the manufacture of a medicament for the treatment and/or the prevention of atherosclerosis.
